# EUROPEAN PATENT SPECIFICATION

(11) **EP 4 527 930 B1**
(45) Date of publication and mention of the grant of the patent: **12.08.2026**
(21) Application number: 24178037.8
(22) Date of filing: 24.05.2024
(51) Int. Cl.: C12N 15/113, A01K 67/0276

(54) **COMPOSITION FOR TREATMENT OF SEPSIS BY REGULATING OLFR164**
ZUSAMMENSETZUNG ZUR BEHANDLUNG VON SEPSIS DURCH REGULIERUNG VON OLFR164
COMPOSITION POUR LE TRAITEMENT DE LA SEPTICÉMIE PAR RÉGULATION DE L'OLFR164

(30) Priority: 21.09.2023 KR 20230126616
(43) Date of publication of application: 26.03.2025
(73) Proprietor: Daegu Gyeongbuk Institute Of Science and Technology, Daegu 42988 (KR); Research & Business Foundation Sungkyunkwan University, Gyeonggi-do 16419 (KR)
(72) Inventor: KOO, Jae Hyung, 42988 Daegu (KR); BAE, Yoe Sik, 13597 Seongnam-si (KR); JUNG, Young Su, 21997 Incheon (KR); JEONG, Yu Sun, 16417 Suwon-si (KR); KIM, Ji Cheol, 16987 Yongin-si (KR); CHOI, Ji Woo, 42988 Daegu (KR)
(74) Representative: J A Kemp LLP

(56) References cited:
- WO-A1-2019/204233
- WO-A2-03/048383
- WO-A2-2019/210268
- JP-A- 2020 112 520
- KR-A- 20090 071 871
- KR-A- 20190 031 751
- SAKURAI TAKAYUKI ET AL: "Production of genetically engineered mice with higher efficiency, lower mosaicism, and multiplexing capability using maternally expressed Cas9", SCIENTIFIC REPORTS, vol. 10, no. 1, 23 January 2020 (2020-01-23), US, XP093223621, ISSN: 2045-2322, Retrieved from the Internet <URL:https://www.nature.com/articles/s41598-020-57996-7> DOI: 10.1038/s41598-020-57996-7

## Description

### BACKGROUND OF THE INVENTION

### 1. Field of the invention

This invention was carried out under the support of the Ministry of Science and ICT of the Republic of Korea, with the project number 2020M3A9D3038435. The research management agency for this project is the National Research Foundation of Korea, the research project title is "Development of BioMedical Technology", the specific research task is "Development of Neutrophil Olfactory Receptor-Based Sepsis Control Technology", the leading institution is Sungkyunkwan University, and the research period was from January 1, 2023 to December 31, 2023.

This application claims priority to and the benefit of Korean Application Number 10-2023-0126616, filed in the Korean Intellectual Property Office on September 21, 2023.

The present disclosure relates to a pharmaceutical composition for use in the prevention or treatment of sepsis, and a method for screening a therapeutic agent for sepsis.

### 2. Description of the Prior Art

Sepsis is a severe acute inflammatory disease in which an immune response is initiated by a pathogen invading the body to excessively secrete inflammatory cytokines and induce a systemic inflammatory response, ultimately resulting in tissue and organ damage, loss of white blood cell function, and paralysis of the body's ability to defend against infection. Among the pathogens, *Pseudomonas aeruginosa* is one of the main bacteria causing sepsis. Infections by *Pseudomonas aeruginosa* can lead to pneumonia, intra-abdominal infections, post-surgical wound infections, osteoarticular infections, and soft tissue infections. *Pseudomonas aeruginosa* is also a common opportunistic pathogen that causes infections when a patient's immune system is weakened.

The treatment for *Pseudomonas aeruginosa* known thus far is achieved through the administration of antibiotics depending on the site of infection. Antibiotics such as polymyxin, levofloxacin, ciprofloxacin, and ceftazidime are commonly used, but serious infections caused by *Pseudomonas aeruginosa* are difficult to treat. Furthermore, the increase of multi-drug resistant *Pseudomonas aeruginosa* that is resistant to many classes of antibiotics (carbapenems, aminoglycosides, fluoroquinolones) has led to the need for alternative methods of controlling *Pseudomonas aeruginosa* infections beyond antibiotic therapy. As of 2017, 14.7% of samples collected from general and long-term care hospitals nationwide were reported to be multi-drug resistant *Pseudomonas aeruginosa.* Due to the nature thereof, *Pseudomonas aeruginosa* quickly acquires resistance even to antibiotics to which it is susceptible, so there are limits to antibiotic treatment.

One of the most notable characteristics of *Pseudomonas aeruginosa* is the opportunistic infection thereof into patients with compromised immune functions. Hence, augmenting and supplementing immune function may be an effective strategy to respond to *Pseudomonas aeruginosa* infections. Immune cells consist of innate immune cells, such as neutrophils, macrophages, and dendritic cells, and adaptive immune cells, such as B cells and T cells. In defending against *Pseudomonas aeruginosa,* innate immune cells play the most crucial role. Immune cells can recognize various substances derived from pathogens and exhibit a defensive response thereto. Immune cells can recognize external inflammatory environments and pathogens through G protein-coupled receptors (GPCRs), which primarily recognize small molecular weight substances as well as through pattern recognition receptors (PRRs), which recognize patterns of pathogens. However, there is little known about which GPCRs are activated by metabolites derived from pathogens and how they regulate functions.

In mammals, the odorant receptor (OR) accounts for over 50% of the GPCRs receptor family. Recent reports have indicated that the physiological functions of odorant receptors are regulated beyond their traditional role in nasal tissues, as they are also expressed in a variety of cells, tissues, and organs. Additionally, there are reports that odorant receptors can recognize metabolites produced by various pathogens, suggesting that odorant receptors selectively resistant to specific pathogens could become an important candidate in future drug development targeting.

WO 2019/204233 A1 describes modulating the immune response via targeting of olfactory receptor activity. WO 2019/210268 A2 describes a cell library for use in detecting protein expression comprising a plurality of cells. Sakuri et al. (2021, Scientific reports, vol. 10, no. 1) describes the production of genetically engineered mice with higher efficiency, lower mosaicism, and multiplexing capability using maternally expressed Cas9. WO03048383 a screening method for identifying an agent that blocks sepsis or inflammation by combining a polynucleotide with an agent that is monitored for modulation of its expression. JP2020112520 is screening for a cat urine odour suppressing compound and identifies OR2M3 (human) antagonists which are homologue to mouse Olfr164. KR20190031751 discloses a pharmaceutical composition for use in the treatmen of leukaemia comprising as active ingredient an olfactory receptor expression or activity inhibitor.

### SUMMARY OF THE INVENTION

The present invention provides a pharmaceutical composition for use in the prevention or treatment of sepsis comprising an Olfr164 (odorant receptor 164) inhibitor; wherein the Olfr164 inhibitor is selected from the Olfr164 expression inhibitors consisting of a single guide RNA (sgRNA), a short interfering RNA (siRNA), a short hairpin RNA (shRNA), a microRNA (miRNA), a ribozyme, and an antisense oligonucleotide, each binding complementarily to the nucleic acid sequence encoding Olfr164.

The present invention also provides a method for screening a therapeutic agent for sepsis comprising: (a) treating a biological sample with a candidate therapeutic substance; comparing the expression level or activity of Olfr164 between samples treated with and without the candidate substance; and determining the candidate substance as a therapeutic agent for sepsis if the expression level or activity of Olfr164 in the sample treated with the candidate substance is decreased compared to the sample treated without the candidate substance.

### DETAILED DESCRIPTION

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes.

According to an aspect thereof, the present disclosure provides a pharmaceutical composition comprising an odorant receptor 164 (Olfr164) inhibitor, wherein the Olfr164 inhibitor is selected from the Olfr164 expression inhibitors consisting of a single guide RNA (sgRNA), a short interfering RNA (siRNA), a short hairpin RNA (shRNA), a microRNA (miRNA), a ribozyme, and an antisense oligonucleotide, each binding complementarily to the nucleic acid sequence encoding Olfr164.

The pharmaceutical composition of the present disclosure may be formulated into powders, granules, tablets, coated tablets, pills, lozenges, capsules, liquids, suspensions, gels, syrups, slurries, suppositories, enemas, emulsions, pastes, ointments, creams, lotions, pulvis, sprays, or suspensions.

The pharmaceutical composition of the present disclosure may further comprise suitable carriers, excipients, or diluents conventionally used in the manufacture of pharmaceutical compositions. Examples include lactose, dextrose, sucrose, sorbitol, mannitol, xylitol, erythritol, starch, acacia gum, alginates, gelatin, calcium phosphate, calcium silicate, cellulose, methylcellulose, amorphous cellulose, polyvinylpyrrolidone, water, methyl hydroxybenzoate, propyl hydroxybenzoate, talc, magnesium stearate, or mineral oil.

The present inventors have diligently researched to develop a method for treating infectious diseases by regulating an odorant receptor (olfactory receptor). As a result, it was discovered that odorant receptor Olfr164 knockout mice secreted cytokines and chemokines at reduced levels and produced reactive oxygen species at an increased level while exhibiting improved survival rates after pathogen infection.

As used herein, the term "odorant receptors" refers to receptors located in the mucous membrane of the nose that detect specific odor molecules and transmit the information to the brain. These receptors are a type of G protein-coupled receptors (GPCRs) and generate olfactory signals in response to various chemical substances. Recent research has revealed that odorant receptors are associated with various diseases. By way of example, studies are being conducted on the relationship between odorant receptors and various diseases including neurodegenerative diseases like Alzheimer's and Parkinson's, respiratory diseases like COVID-19, and metabolic and endocrine-related diseases like obesity and diabetes. However, the precise functioning manner of odorant receptors and their mechanisms in disease development are not yet fully understood, indicating a need for further research.

As used herein, the term "odorant receptor Olfr164", also known as MOR279-2, refers to a protein consisting of 315 amino acids, encoded by a gene (SEQ ID NO: 3) located on chromosome 16 A3 in mice.

As used herein, the term "cytokine" refers to small proteins used in signaling among cells that are part of the immune system and play an important role in various biological responses such as infection, inflammation, and immune reactions.

As used herein, the term "sepsis" refers to a symptom that occurs due to bacterial or viral infections, severe trauma, etc., and induces excessive activation of the body's self-defense system, such as macrophages and cytokine storms. This excessive systemic inflammatory response can cause circulatory abnormalities, increase vascular permeability due to capillary leakage, lower blood pressure, and eventually lead to insufficient blood supply to various organs, resulting in multiple organ failure (MOF). Sepsis is a representative intractable disease with a mortality rate of about 30%, affecting approximately 27 million people worldwide each year. It is the third leading cause of death globally, following cancer and heart disease. Therefore, the goal of sepsis treatment is to suppress the body and cellular inflammatory response and block the cytokine storm.

In an embodiment of the present disclosure, the sepsis is caused by a virus, a bacterium, a fungus, a parasite, a protozoon, a hemorrhagic infectious agent, or a combination thereof.

In an embodiment of the present disclosure, the sepsis is bacterial sepsis. As used herein, the term "bacterial sepsis" refers to sepsis caused by bacterial infection. The bacteria causing sepsis may be exemplified by Staphylococcus aureus, Escherichia coli, Streptococcus pneumoniae, *Pseudomonas aeruginosa,* or Enterococcus, but are not limited thereto.

In an embodiment of the present disclosure, the bacteria may be *Pseudomonas aeruginosa. Pseudomonas aeruginosa* is an aerobic Gram-negative bacillus commonly found in the natural environment and does not form spores. *Pseudomonas aeruginosa* can infect plants, animals, and humans. When infected with *Pseudomonas aeruginosa,* healthy individuals may not be severely harmed, but people with cystic fibrosis or compromised immune systems may undergo serious symptoms.

As used herein, the term "cytokine storm" refers to a dysregulation of pro-inflammatory cytokines that causes diseases known as "cytokine release syndrome" or "inflammatory cascade". A cytokine storm or cascade often occurs because one cytokine can induce the production of several other cytokines that generally augment and amplify the immune response.

As used herein, the term "prevention" refers to a prophylactic or protective treatment of a disease or a disease condition. As used herein, the term "treatment" refers to a reduction, suppression, amelioration, or eradication of a disease condition. For example, treatment means minimizing the spread or worsening of a disease by administering a therapeutic agent to a subject.

As used herein, the terms "subject" or "patient" refer to an animal, preferably a mammal, including humans, pigs, chimpanzees, dogs, cats, cattle, mice, rabbits, or rats.

In the present disclosure, the Olfr164 (odorant receptor 164) inhibitor or the pharmaceutical composition containing same is administered in a pharmaceutically effective amount. Herein, "pharmaceutically effective dose" means an amount sufficient to treat disease at a reasonable benefit/risk ratio applicable to any medical treatment, and the effective dose level may be determined depending on various factors including the patient's disease type, severity, drug activity, drug sensitivity, administration time, administration route and excretion rate, treatment period and concomitant drugs, and other factors well known in the medical field.

Specifically, the effective dose of the Olfr164 (odorant receptor 164) inhibitor or the pharmaceutical composition containing same may vary depending on administration routes, severity of disease, patient's age, sex, body weight, and age. Therefore, the dose does not limit the scope of the present disclosure in any way.

The Olfr164 (odorant receptor 164) inhibitor or the pharmaceutical composition containing same may be administered preferably at a dose of 0.001 to 100 mg/kg (body weight) a day. The dose of the pharmaceutical composition may be topically applied to desired sites according to purposes.

The Olfr164 (odorant receptor 164) inhibitor or a pharmaceutical composition containing same according to the present disclosure may be administered to a subject via various routes. All modes of administration are contemplated. Examples include administration through oral, sublingual, buccal mucosa, and dermal routes, injection through subcutaneous, intraperitoneal, intravenous, intramuscular, epidural (intradural), intraocular, auricular, intranasal, and transdermal routes, insertion through intrarectal and intravaginal routes, inhalation, and spray through the mouth and nose.

The Olfr164 (odorant receptor 164) inhibitor or the pharmaceutical composition containing same according to the present disclosure may be administered as an individual therapeutic agent or in combination with other therapeutic agents. In the latter case, the receptor may be administered sequentially or simultaneously with conventional therapeutic agents, either as a single or multiple doses. It is important to administer an amount that maximizes efficacy with minimal side effects, considering all the aforementioned factors. This amount can be readily determined by a person skilled in the art to which the present disclosure pertains.

The Olfr164 inhibitor is selected from the Olfr164 expression inhibitors consisting of a single guide RNA (sgRNA), a short interfering RNA (siRNA), a short hairpin RNA (shRNA), a microRNA (miRNA), a ribozyme, and an antisense oligonucleotide, each binding complementarily to the nucleic acid sequence encoding Olfr164.

As used herein, the term "inhibition of the expression of Olfr164 (odorant receptor 164)" refers to a situation where the quantitative expression value of the Olfr164 gene or protein in the test group is measurably reduced compared to the control group, as exemplified by the cases where the expression level of the Olfr164 gene or protein in the test group is 90% or less, 80% or less, or 70% or less compared to the control group, but with no limitations thereto.

The term "gene expression", as used herein, is intended to encompass the transcription into polynucleotides and translation into polypeptides of genes and the modification of the polynucleotide and/or polypeptides (e.g., post-translational modifications of the polypeptides). An "expressed gene" refers to a gene transcribed into mRNA and then translated into a polypeptide, or transcribed into RNA but not translated into a polypeptide (e.g., tRNA and rRNA).

As used herein, the term "single guide RNA" (sgRNA) refers to an RNA that is a crucial component of the CRISPR-Cas9 gene-editing system. sgRNA directs the Cas9 protein to a specific target location on a gene, where the Cas9 protein then cuts the DNA at that specific location. sgRNA consists of two main components. The CRISPR RNA (crRNA) determines the site of DNA which the Cas9 protein will cleave. The crRNA is designed to match a specific DNA sequence of the target. The trans-activating crRNA (tracrRNA) interacts with the Cas9 protein to help the crRNA bind to the Cas9 protein.

As used herein, the term "microRNA" (miRNA) refers to a short non-coding RNA molecule approximately 22 nucleotides in length. The RNA molecules are known to function as a posttranscriptional regulator in the gene expression process. They bind to target mRNAs with sequences complementary thereto, thus degrading the target mRNAs or inhibiting their translation into proteins.

As used herein, the term "short interfering RNA" (siRNA) refers to a short double-stranded RNA molecule that can specifically act on a particular gene and induce RNA interference (RNAi) by cleaving the mRNA thereof. siRNA can be used as an efficient method for gene knockdown or in gene therapy as it can suppress the expression of the target gene. siRNA targeting a gene consists of a sense RNA strand that includes a sequence homologous to all or part of the target gene nucleic acid sequence and an antisense RNA strand that includes a complementary sequence thereto, and thus may include a nucleotide sequence that hybridizes with the mRNA of the target gene in the cell. The siRNA is not limited to fully paired double-stranded RNA parts, but may include unpaired parts due to mismatches where the corresponding bases are not complementary or one strand lacks a corresponding base. The total length may be 10 to 100 nucleotides, preferably 10 to 80 nucleotides, more specifically 10 to 70 nucleotides, and even more specifically 10 to 30 nucleotides. The siRNA molecules may have a short nucleotide sequence, for example, about 5-15 nt long, inserted between self-complementary sense and antisense strands. In this regard, the siRNA molecules generated by expression of the nucleotide sequences form hairpin structures upon intramolecular hybridization, appearing as an overall stem-and-loop structure. This stem-and-loop structure can be processed in vitro or in vivo to generate active siRNA molecules that mediate RNAi.

As used herein, the term "short hairpin RNA" (shRNA) refers to RNA molecules having an RNA sequence that makes a tight hairpin turn that can be used to silence gene expression via RNA interference. The shRNA hairpin structure is cleaved by the cellular machinery into siRNA, which is then bound to the RNA-induced silencing complex (RISC). This complex binds to and cleaves mRNAs which match the siRNA that is bound to it. The sequence of the siRNA can correspond to the full-length target gene, or a subsequence thereof. siRNA is "targeted" to a gene in that the nucleotide sequence of the duplex portion of the siRNA is substantially complementary to a nucleotide sequence of the targeted gene. The siRNA sequence duplex needs to be of sufficient length to bring the siRNA and target RNA together through complementary base-pairing interactions. The siRNA of the present disclosure may be of varying lengths. The length of the siRNA is preferably greater than or equal to ten nucleotides and of sufficient length to stably interact with the target RNA; specifically 10-30 nucleotides; more specifically any integer between 10 and 30 nucleotides, such as 10, 11, 12, 13, 14, 15, 16, 17, 18, 19, 20, 21, 22, 23, 24, 25, 26, 27, 28, 29, and 30. By "sufficient length" is meant a nucleotide of greater than or equal to 10 nucleotides that is of a length great enough to provide the intended function under the expected condition. The shRNA may be cloned into a vector using recombinant DNA techniques.

As used herein, the term "ribozyme" refers to RNA molecules with catalytic activity. Various ribozymes different in activity are known, and ribozymes with selective target-specific RNA cleavage activity can be manufactured by standard techniques known in the art.

The term "antisense nucleotide", as used herein, refers to an oligomer or polymer of nucleotide or nucleoside monomers composed of naturally occurring sugars, nucleobases, and intersugar linkages. Also, the term is intended to encompass modified or substituted nucleotide oligomer having non-naturally occurring monomers or moieties thereof which function similarly. The incorporation of such substituted oligomers is based on factors including enhanced cellular uptake and increased stability in the presence of nucleases and can be selected in a manner well known in the art. The oligonucleotide may be substituted entirely or partially. In addition, the antisense oligonucleotides of the present disclosure may include oligomer mimetics modified by methods well known in the art, such as peptide nucleic acids (PNA) and locked nucleic acids (LNA), to increase the affinity of an oligonucleotide for its target and provide tolerance for mismatches to the target sequence. Also, examples of the antisense oligonucleotides include native oligonucleotides, phosphorothioate oligodeoxyribonucleotide, phosphorodithioate oligodeoxyribonucleotide, methylphosphonate oligodeoxyribonucleotide, phosphoramidate oligodeoxyribonucleotide, H-phosphonate oligodeoxyribonucleotide, triester oligodeoxyribonucleotide, alpha-anomeric oligodeoxyribonucleotide, peptide nucleic acids, and modified oligonucleotides such as artificial nucleic acids and nucleic acid-modified compounds, but are not limited thereto. Antisense oligonucleotide molecules that bind to the Olfr164 gene or sequences complementary to the Olfr164 gene can be isolated or manufactured using standard molecular biology techniques, such as chemical synthesis or recombinant methods, or can be obtained commercially.

Also described herein is an agent that specifically binds to or reacts with the Olfr164 protein to inhibit or suppress its activity.

An agent capable of inhibiting activity refers to a substance that decreases the activity or function of a protein. Herein, the agent may be a compound, peptide, peptide mimetic, aptamer, antibody, or antigen-binding fragment of an antibody, or a combination thereof, that specifically binds to the Olfr164 protein. By way of example, it may include antibodies such as polyclonal, monoclonal, or recombinant antibodies that bind to the Olfr164 protein. Additionally, the term "peptide mimetics" refer to compounds that mimic the structure and desirable characteristics of a specific polypeptide for therapeutic purposes.

As used herein, the term "antibody" refers to a substance that specifically binds to an antigen and induces an antigen-antibody reaction. Herein, the antibody refers to one that specifically binds to a protein encoded by the aforementioned gene and can be produced according to conventional methods in the field. The antibody may be in the form of a polyclonal, monoclonal, or recombinant antibody. Antibodies can be easily produced using widely known techniques in the field.

As used herein, the term "aptamer" refers to a polynucleotide composed of a special type of single-stranded nucleic acid (DNA, RNA, or modified nucleic acid) that forms a stable tertiary structure as it is and has the characteristic of binding to a target molecule with high affinity and specificity. As stated in the foregoing, aptamers, like antibodies, can specifically bind to antigenic substances while being composed of polynucleotides which are more stable, have simpler structures, and are easier to synthesize than proteins. Therefore, aptamers can be used as alternatives to antibodies.

In an embodiment, the Olfr164 (odorant receptor 164) inhibitor is an sgRNA comprising a nucleotide sequence having at least 80%, 85%, 90%, or 95% identity to SEQ ID NO: 5.

In an embodiment, the Olfr164 (odorant receptor 164) inhibitor is sgRNA comprising or composed of SEQ ID NO: 5.

In an embodiment of the present disclosure, the pharmaceutical composition inhibits the expression of IL-6, TNF-α, IL-1β, IL-10, or a combination thereof. The cytokines are not limited to IL-6, TNF-α, IL-1β, IL-10, or a combination thereof, but may include IFN-γ, etc.

As used herein, the term "tumor necrosis factor-alpha" (TNF-α) refers to one of the main cytokines promoting inflammatory responses, which exhibits an increased expression level in sepsis. TNF-α can induce a stronger inflammatory response by increasing the production of other cytokines. "Interleukin-1" (IL-1) is another powerful pro-inflammatory cytokine that, like TNF-α, worsens inflammation by increasing the production of other cytokines. "Interleukin-6" (IL-6) is a pro-inflammatory cytokine involved in immune responses and inflammation. "Interferon gamma" (IFN-gamma) is primarily produced by Th1 subtype T cells and activates destructive immune responses immediately. "IL-10" is an anti-inflammatory cytokine and an immunosuppressive cytokine produced by various mammalian cell types including macrophages, monocytes, T cells, B cells, and keratinocytes.

An aspect of the present disclosure provides a method for screening a sepsis therapeutic agent comprising:
(a) treating a biological sample with a candidate therapeutic substance;
(b) comparing the expression level or activity of Olfr164 between the samples treated with and without the candidate substance; and
(c) determining the candidate substance as a therapeutic agent for sepsis if the expression level or activity of Olfr164 in the sample treated with the candidate substance is decreased compared to the sample treated without the candidate substance.

In an embodiment of the present disclosure, the biological sample is at least one selected from blood, plasma, serum, saliva, nasal fluid, sputum, synovial fluid, amniotic fluid, ascites, cervical or vaginal secretions, urine, and cerebrospinal fluid of the subject. Preferably, the method is an *ex vivo* method.

Also described herein is a transgenic mouse with the Olfr164 gene knocked out.

As used herein, the term "knockout" refers to the manipulation of the DNA sequence of a target gene so as to prevent the gene from properly producing its protein. When knocked out, a gene loses its function or greatly decreases in function.

In a specific embodiment of the present disclosure, the relationship between the Olfr164 and the expression of cytokines or chemokines was elucidated. Therefore, the transgenic mouse with the Olfr164 gene knocked out can be used to study how specific environmental factors alter biological responses in the knockout state of the Olfr164 gene. Moreover, the interactions between the gene and other internal factors in the body can be studied by observing and analyzing the overall physiological and pathological changes that occur in the absence of the Olfr164 gene.

In an embodiment of the present disclosure, the knockout of the Olfr164 gene comprises the deletion of the sequence of 11 nucleotides stretching from nucleotides 246 to 256 in the nucleic acid sequence of SEQ ID NO: 3 encoding Olfr164. The Olfr164 gene that was knocked out is shown in SEQ ID NO: 4.

The features and advantages of this invention can be summarized as follows:
(a) The present disclosure provides a pharmaceutical composition for use in the prevention or treatment of sepsis comprising an Olfr164 (odorant receptor 164) inhibitor; and
(b) The present disclosure provides a method for screening a sepsis therapeutic agent, the method comprising the steps of treating a biological sample with a candidate therapeutic substance and comparing the expression level or activity of Olfr164 in the treated sample to that in an untreated sample, preferably wherein the method is an *ex vivo* method.

### BRIEF DESCRIPTION OF THE DRAWINGS

The above and other aspects, features and advantages of the present disclosure will be more apparent from the following detailed description taken in conjunction with the accompanying drawings, in which:
FIG. 1 illustrates the knockout location of the gene encoding Olfr164 in the creation of Olfr164 knockout mice. The knockout of 11 base pairs within the Olfr164 transcription site was achieved using the CRISPR/CAS9 system, and the knockout was verified using sequencing and the T7E1 system;
FIG. 2 shows the results of genotyping after the creation of Olfr164 knockout mice, confirming the production of a shorter DNA fragment in mice with 11 base pairs knocked out;
FIG. 3 compares the survival rates of Wild Type (WT) mice and Olfr164 knockout mice after infection with the PAO1 strain of *Pseudomonas aeruginosa;*
FIG. 4 compares the secretion levels of inflammatory cytokines IL-6, TNF-α, IL-1β, and IL-10 in the peritoneal fluid 12 hours after infecting WT and Olfr164 knockout mice with *Pseudomonas aeruginosa;*
FIG. 5 shows the secretion levels of chemokines CCL2, CXCL1, and CXCL2 in the peritoneal fluid 12 hours after infecting WT and Olfr164 knockout mice with *Pseudomonas aeruginosa;*
FIG. 6 shows the secretion levels of inflammatory cytokines IL-6, TNF-α, and chemokines CCL2, CXCL1 in peripheral blood 12 hours after infecting WT and Olfr164 knockout mice with *Pseudomonas aeruginosa;*
FIG. 7 compares the counts of *Pseudomonas aeruginosa* in major organs after infection in WT and Olfr164 knockout mice.
FIG. 8 shows the results of comparing superoxide production after activating bone marrow-derived neutrophils from WT and Olfr164 knockout mice with *Pseudomonas aeruginosa;*
FIG. 9 compares the expression levels of cytokines after treating bone marrow-derived neutrophils from WT and Olfr164 knockout mice with LPS (lipopolysaccharide) derived from *Pseudomonas aeruginosa;* and
FIG. 10 compares the survival rates against *Pseudomonas aeruginosa* infection after adoptively transferring neutrophils from wild-type mice and Olfr164-deficient neutrophils into neutrophil-depleted wild-type mice.

### DETAILED DESCRIPTION OF THE EXEMPLARY EMBODIMENTS

A better understanding of the present disclosure may be obtained through the following Examples, which are set forth to illustrate, but are not to be construed to limit the present disclosure.

### EXAMPLES

### EXAMPLE 1: Construction and Verification of Olfr164 Knockout Mice

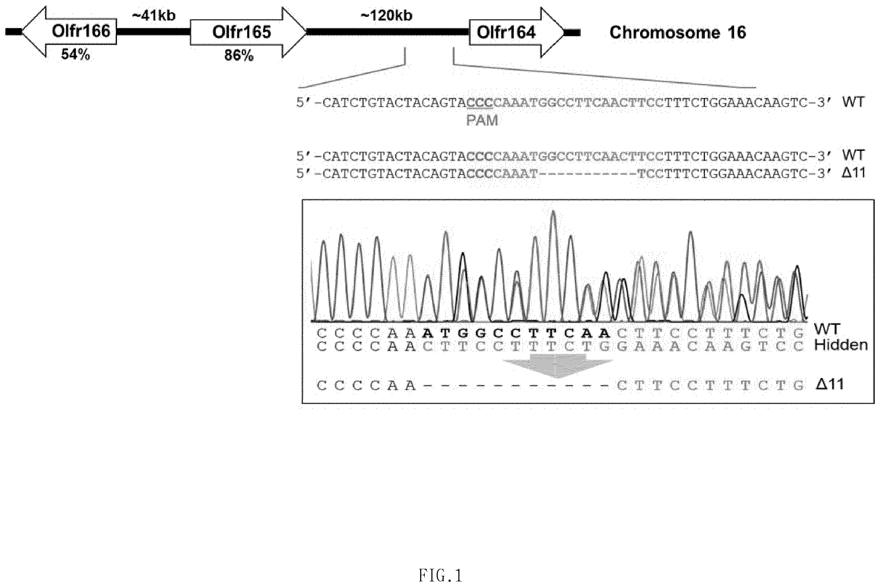

To examine the effects of Olfr164 knockout on *Pseudomonas aeruginosa* infection, knockout mice for in vivo experiments were custom-ordered through GH Bio. In brief, microinjection was performed on frozen embryos of C57BL/6 mice, and a mixture of Olfr164 sgRNA (100 ng/µl; SEQ ID NO: 5) and Cas9 protein (80 ng/µl) was injected into the one-cell stage embryos. These injected embryos were then implanted into surrogate mothers of the ICR strain the next day. The location and base pair deletion of Olfr164 knockout within the gene are shown in FIG. 1.

Genotyping was performed via PCR to confirm the deletion of Olfr164. Mouse tails were incubated in genomic DNA extraction buffer and 250ug/ml proteinase K (#P2308, Sigma-Aldrich) at 60°C for 16 hours to obtain DNA which was then diluted in sterile distilled water. The sequences of the primers are as follows: forward; 5'-CCA TGT ACA TCC TTC TCA GC-3' (SEQ ID NO: 1), Reverse; 5'-GAA GAA TAT CTG GAT TCC ACA G-3' (SEQ ID NO: 2). The PCR cycle for genotyping was as follows: starting at 94°C for 5 minutes and then, 38 cycles (94°C for 30 seconds, 62.5°C for 30 seconds, 72°C for 30 seconds), followed by 72°C for 10 minutes. To confirm the 11-bp deletion, electrophoresis was performed for 25 minutes on a 4% agarose gel.

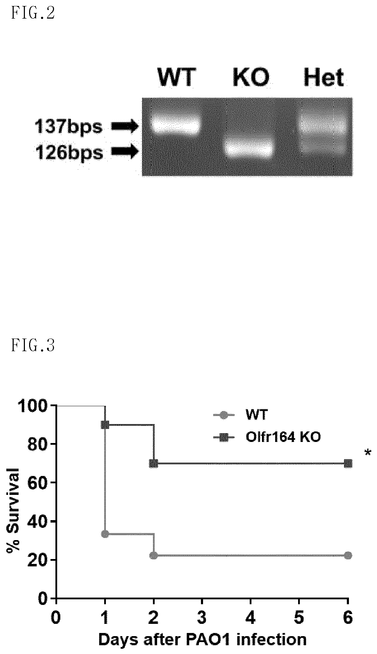

The results are depicted in FIG. 2.

As shown in FIG. 2, a nucleic acid fragment 11-bp shorter was found in the knockout mice, demonstrating that mice with the specific 11-base pair knocked out Olfr164 gene were successfully created through the CRISPR/Cas9 system.

### EXAMPLE 2: Assay for Function of Olfr164 in Pseudomonas aeruginosa-Infected Model

### 2-1. Preparation and Culturing of Pseudomonas aeruginosa

*Pseudomonas aeruginosa* (PAO1 strain) was cultured on Bacto tryptic soy agar (TSA media, #236950; BD) and Difco tryptic soy broth (TSB medium, #211825; BD). Initially, *Pseudomonas aeruginosa* was cultured on TSA plates at 37°C for 16 hours. Subsequently, the grown bacteria were transferred to TSB broth and cultured at 37°C in a shaking incubator at 220 rpm for 14 hours. The cultured *Pseudomonas aeruginosa* was centrifuged at 3000 X rpm for 5 minutes, washed twice with PBS (phosphate buffered saline), and then counted by reading the absorbance. For animal injection experiments, bacteria were injected intraperitoneally (I.P.) at a dose of 3X10⁷/200*µ*ℓ/head to animals

### 2-2. Preparation of Animal Models

All mice were maintained and managed in an environment and protocol controlled at standard temperatures and approved by the Animal Care and Ethics Committee of Sungkyunkwan University. Only male mice aged between 8-10 weeks were used for both the in vivo infection animal model and in vitro neutrophil isolation experiments.

### 2-3. Survival Rate Comparison between Olfr164 Knockout and Wild-Type Mice

Although there are various infection routes for *Pseudomonas aeruginosa,* intraperitoneal injection was performed as it is a representative cause leading to sepsis. After injecting *Pseudomonas aeruginosa* (3X10⁷ cells) into the peritoneal cavity of mice, the survival rate difference based on the absence or presence of Olfr164 was observed. The survival of mice was checked every 12 hours after injection.

The results are depicted in FIG. 3.

As shown in FIG. 3, a significant difference in the survival rate between Olfr164 wild-type and knockout mice was observed within 24 hours after *Pseudomonas aeruginosa* injection. While Olfr164 wild-type mice showed a mortality rate of about 70% within 24 hours, the survival rate of mice with Olfr164 knocked out was significantly higher (70% survival rate).

### 2-4. Assay for Inflammatory Cytokine and Chemokine Secretion Levels

To specifically elucidate the cause of the difference in survival rates between wild-type and knockout mice after *Pseudomonas aeruginosa* administration, both wild-type and knockout mice were injected intraperitoneally with *Pseudomonas aeruginosa* at the same dose (3X10⁷ cells). Twelve hours post-injection, peripheral blood and peritoneal fluid were collected to compare cytokine and chemokine secretion levels.

The cytokine expression levels in samples (peritoneal lavage fluid and blood) collected from the animal model were measured using an ELISA kit according to the manufacturer's protocols. The ELISA kit was purchased from Thermo Scientific (IL-6 #88-7064-88, TNFα #88-7324-88, IL-1β #88-7013A-88, IL-10 #88-7105-88, CCL2 #88-7391-88) and RnD Systems (CXCL1 #DY453, CXCL2 #DY452). Sample collection and preparation from the animal model were as follows. For peritoneal lavage fluid, 2ml of PBS was injected into the peritoneum to thoroughly wash the inside and then withdrawn with a syringe. After centrifugation (12000 rpm, 1 minute) to separate cells and lavage fluid, only the lavage fluid was used for ELISA. Blood was collected by orbital bleeding, placed in a tube containing EDTA, and centrifuged (6000 rpm, 10 minutes) to obtain plasma for use in ELISA.

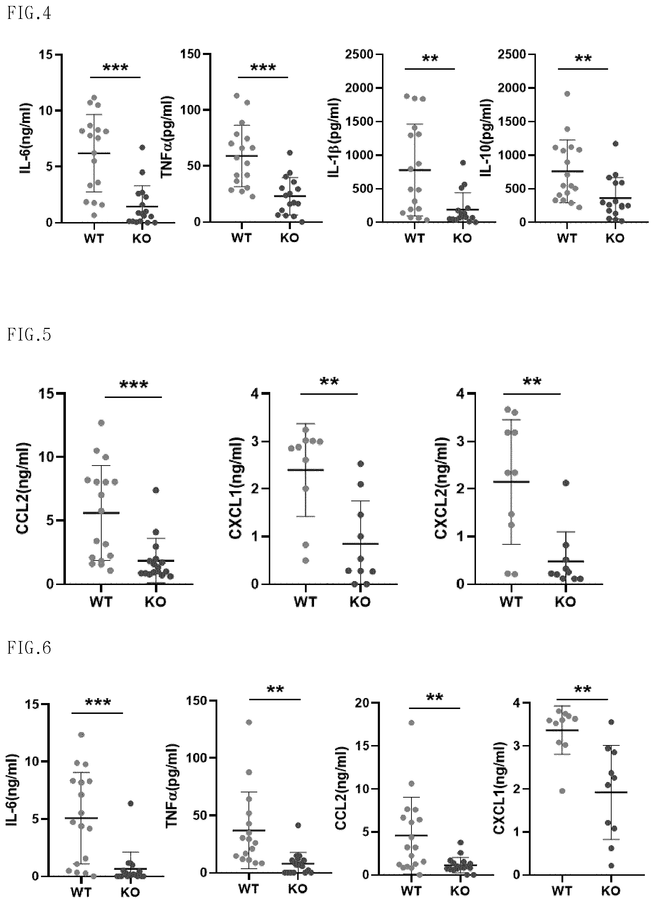

The results are depicted in FIGS. 4, 5, 6, and 7.

As shown in FIG. 4, the secretion levels of inflammatory cytokines (IL-6, TNF-α, IL-1β, IL-10) in the peritoneal fluid were found to decrease by 50 to 70% in knockout mice with statistical significance(P<0.01), compared to wild-type mice.

As shown in FIG. 5, the secretion levels of chemokines (CCL2, CXCL1, CXCL2) that induce immune cell migration in the peritoneal fluid, the site of bacterial injection, were found to decrease by 60 to 70% in Olfr164 knockout mice compared to wild-type mice.

As shown in FIG. 6, the secretion levels of cytokines and chemokines in peripheral blood, indicating systemic response outside the site of inflammation, were found to decrease by 40 to 80% in Olfr164 knockout mice compared to wild-type mice. The decrease in inflammatory cytokines and chemokines signifies that Olfr164 knockout can increase survival rate during bacterial infection by reducing the cytokine storm, which can cause sepsis due to organ damage.

### 2-5. Comparison of Pseudomonas aeruginosa Counts in Major Organs after Infection

To specifically elucidate the cause of the difference in survival rates between wild-type and knockout mice after *Pseudomonas aeruginosa* administration, comparison was made of the bactericidal effects according to Olfr164 knockout. The same concentration of *Pseudomonas aeruginosa* (3X10⁷ cells) was injected intraperitoneally into both wild-type and knockout mice, and after 12 hours, peritoneal fluid, lung, liver, kidney, and spleen samples were collected to check *Pseudomonas aeruginosa* counts. The prepared samples were diluted in PBS to an appropriate concentration, then spread onto TSA plates, and cultured in a 37°C incubator for 16 hours. The colonies grown on the plates were counted to calculate the colony count in the original samples. For the mouse infection model, lungs, liver, kidneys, and spleen were collected 12 hours after bacterial injection and homogenized in 1ml of PBS to prepare samples.

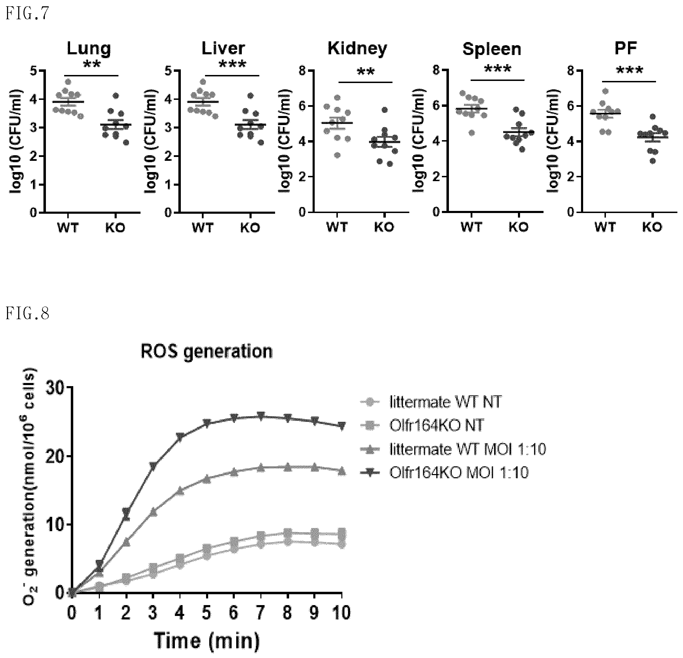

The results depicted in FIG. 7.

As shown in FIG. 7, the Olfr164 knockout mice were found to effectively control bacterial counts compared to wild-type mice, implying that Olfr164 plays a role in controlling *Pseudomonas aeruginosa* not only at the site of infection but also systemically.

Consequently, Olfr164 knockout significantly enhances resistance to *Pseudomonas aeruginosa,* increasing survival rates. This was confirmed to be due to the reduction in bacterial counts and the decrease in the secretion of inflammatory cytokines and chemokines related to cytokine storms, which are causes of organ damage.

### EXAMPLE 3: Assay for Reactivity to Pseudomonas aeruginosa in Wild-Type and Olfr164 Deficient Neutrophils

### 3-1. Isolation of Bone Marrow-Derived Neutrophils

In this example, neutrophils derived from mouse bone marrow were isolated for in vitro experiments. In brief, femur and tibia of mice were collected, and bone marrow was extracted using PBS in a syringe. The collected bone marrow was then centrifuged at 400 x g for 5 minutes at room temperature to harvest cells. The cells were resuspended in 1 ml of HBSS (#LB 003-03; Welgene) and layered on top of a Percoll (#17-0891-01; Cytiva) gradient in a tube. The Percoll gradient was made by diluting 47%, 60%, and 80% Percoll in HBSS. Neutrophils, along with red blood cells, were positioned between the 47% and 60% Percoll layers. Red blood cells were lysed using ACK lysis buffer (#A1049201; Gibco) at room temperature for 8 minutes. The isolated neutrophils were resuspended in RPMI1640 medium until use for experiments.

### 3-2. Comparison of Reactive Oxygen Species Production in Response to Pseudomonas aeruginosa in Wild-type and Olfr164 Knockout Neutrophils

The reactivity of neutrophils to *Pseudomonas aeruginosa* was compared between neutrophils from the wild-type and knockout animals. The production of reactive oxygen species (ROS) is one of the primary bactericidal actions of neutrophils. When generated by external stimuli, ROS is used by neutrophils as signaling molecules for granule release processes and formation of neutrophil extracellular traps (NETs) necessary for bactericidal performance, or ROS itself can act as a bactericidal agent. The amount of ROS released by neutrophils was measured by assessing the reduction of cytochrome C. Neutrophils (1X10⁶ cells) were activated with *Pseudomonas aeruginosa* at a multiplicity of infection (MOI) of 10, and the superoxide production in neutrophils from Olfr164 knockout mice was compared to that from wild-type mice. The concentration of superoxide was measured using a 96-well microplate reader. Specifically, wild-type/Olfr164 knockout neutrophils were prepared at 1.0X10⁶ cells/well and treated with Cytochalasin B (5uM; #C6762; Sigma Aldrich) at room temperature for 5 minutes. The concentration of ROS was measured by the reduction of cytochrome C (50uM; #C2037; Sigma Aldrich). The change in optical density (OD) at 560 nm was measured and calculated at 1-minute intervals for 10 minutes.

The results are depicted in FIG. 8.

As shown in FIG. 8, when assessing the production of ROS over 10 minutes by measuring the reduction of cytochrome C at 1-minute intervals, a difference in ROS production between wild-type and Olfr164 knockout neutrophils was observed from the beginning (2 minutes). It was found that Olfr164 knockout neutrophils had approximately 30 to 50% higher superoxide production upon *Pseudomonas aeruginosa* infection compared to wild-type mice.

### 3-3. Comparison of Cytokine Production in Olfr164 Wild-Type and Knockout Neutrophils in Response to Pseudomonas aeruginosa-Derived LPS

As observed in the animal model through *Pseudomonas aeruginosa* infection (FIGS. 4, 5, and 6), one reason for the increased survival rate in Olfr164 knockout mice can be attributed to the reduction of the cytokine storm. This function was further verified in vitro to see if it was reproduced in neutrophils, which are immune cells. To check cytokine production, neutrophils isolated from bone marrow were activated with 100ng/ml LPS (lipopolysaccharide) derived from *Pseudomonas aeruginosa.* The treatment of neutrophils with LPS was carried out in RMPI1640 containing 2% FBS, and the reaction was conducted in a 37°C incubator for 24 hours. Subsequently, the supernatant was collected and cytokine levels were measured via ELISA.

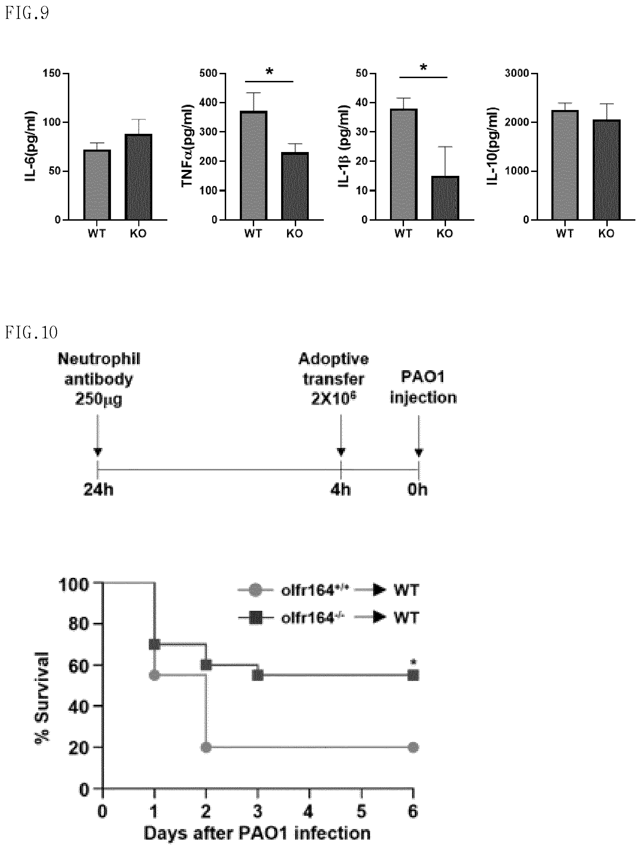

The results are depicted in FIG. 9.

As shown in FIG. 9, while there was no significant difference in the levels of IL-6 and IL-10 between wild-type neutrophils and Olfr164 knockout neutrophils, a significant (P<0.05) decrease of 40 to 60% was observed in TNF-α and IL-1β levels in Olfr164 knockout neutrophils. This suggests that in the response of neutrophils to *Pseudomonas aeruginosa,* Olfr164 knockout neutrophils produce fewer cytokines, potentially leading to the suppression of subsequent excessive immune responses.

### EXAMPLE 4: Assay for Resistance to Pseudomonas aeruginosa Infection through Adoptive Transfer of Wild-type and Olfr164 Knockout Neutrophils

As the presence or absence of Olfr164 in neutrophils, which are crucial immune cells in defending against *Pseudomonas aeruginosa* infection, was found to affect the reactivity in Example 3, examination was made to see whether adoptive transfer of neutrophils in vivo alters defense capabilities.

To conduct experiments under identical conditions, 20 hours before adoptive transfer of neutrophils, anti-Ly6G antibody (Clone: 1A8; #BE0075-1; BioXcell) at 250µg was intraperitoneally injected to deplete neutrophils in wild-type mice. Four hours before infecting with *Pseudomonas aeruginosa* intraperitoneally, neutrophils were isolated from both wild-type and Olfr164 knockout mice using an isolation kit (#480058; Biolegend), and neutrophils (2X10⁶ cells) from either wild-type or knockout mice were injected intravenously into the tail vein of neutrophil-depleted wild-type mice. *Pseudomonas aeruginosa* was injected intraperitoneally at 3.5X10⁶ CFU per mouse, and the survival rate was observed at 12-hour intervals for 6 days post-infection.

The results are depicted in FIG. 10.

As shown in FIG. 10, mice injected with wild-type neutrophils showed a survival rate of 56% within 24 hours and 20% within 48 hours post-infection. In contrast, mice injected with Olfr164 knockout neutrophils maintained a 60% survival rate even at 72 hours. This demonstrates that the introduction of even a single type of immune cell can improve the defense capabilities against *Pseudomonas aeruginosa* infection.

## Claims

1. A pharmaceutical composition for use in the prevention or treatment of sepsis comprising an Olfr164 (odorant receptor 164) inhibitor:
wherein the Olfr164 inhibitor is selected from the Olfr164 expression inhibitors consisting of a single guide RNA (sgRNA), a short interfering RNA (siRNA), a short hairpin RNA (shRNA), a microRNA (miRNA), a ribozyme, and an antisense oligonucleotide, each binding complementarily to the nucleic acid sequence encoding Olfr164.

2. The pharmaceutical composition for use of claim 1, wherein the Olfr164 (odorant receptor 164) inhibitor is an sgRNA comprising a nucleotide sequence having at least 80%, 85%, 90%, or 95% identity to SEQ ID NO: 5.

3. The pharmaceutical composition for use of any one of claims 1 to 2, wherein the Olfr164 (odorant receptor 164) inhibitor is an sgRNA comprising or composed of SEQ ID NO: 5.

4. A method for screening a therapeutic agent for sepsis comprising:
(a) treating a biological sample with a candidate therapeutic substance;
(b) comparing the expression level or activity of Olfr164 between samples treated with and without the candidate substance; and
(c) determining the candidate substance as a therapeutic agent for sepsis if the expression level or activity of Olfr164 in the sample treated with the candidate substance is decreased compared to the sample treated without the candidate substance.

5. The method of claim 4, wherein the biological sample is at least one selected from the group consisting of blood, plasma, serum, saliva, nasal fluid, sputum, synovial fluid, amniotic fluid, ascites, cervical or vaginal secretions, urine, and cerebrospinal fluid of the subject.

## Patentansprüche

1. Pharmazeutische Zusammensetzung für die Verwendung bei der Vorbeugung oder Behandlung von Sepsis, die einen Olfr164- (Geruchsrezeptor 164) Inhibitor umfasst:
wobei der Olfr164-Inhibitor aus den Olfr164-Expressionsinhibitoren, bestehend aus einer Single Guide RNA (sgRNA), einer kurzen interferierenden RNA (siRNA), einer kurzen Haarnadelstruktur-RNA (shRNA), einer microRNA (miRNA), einem Ribozym und einem Antisense-Oligonukleotid, die jeweils komplementär an die Nukleinsäuresequenz binden, die für Olfr164 kodiert.

2. Pharmazeutische Zusammensetzung für die Verwendung nach Anspruch 1, wobei der Olfrl64- (Geruchsrezeptor 164) Inhibitor eine sgRNA ist, die eine Nukleotidsequenz umfasst, die wenigstens 80 %, 85 %, 90 % oder 95 % Identität zu SEQ ID NO: 5 aufweist.

3. Pharmazeutische Zusammensetzung für die Verwendung nach einem der Ansprüche 1 bis 2, wobei der Olfrl64- (Geruchsrezeptor 164) Inhibitor eine sgRNA ist, die SEQ ID NO: 5 umfasst oder aus dieser besteht.

4. Verfahren zum Screening eines Therapeutikums gegen Sepsis, umfassend:
(a) Behandeln einer biologischen Probe mit einer therapeutischen Kandidatensubstanz;
(b) Vergleichen des Expressionsniveaus oder der Aktivität von Olfr164 zwischen Proben, die mit und ohne die Kandidatensubstanz behandelt wurden; und
(c) Bestimmen der Kandidatensubstanz als ein Therapeutikum gegen Sepsis, wenn das Expressionsniveau oder die Aktivität von Olfr164 in der mit der Kandidatensubstanz behandelten Probe im Vergleich zu der ohne die Kandidatensubstanz behandelten Probe verringert ist.

5. Verfahren nach Anspruch 4, wobei die biologische Probe wenigstens eine ist, die aus der Gruppe ausgewählt ist, bestehend aus Blut, Plasma, Serum, Speichel, Nasenflüssigkeit, Sputum, Synovialflüssigkeit, Fruchtwasser, Aszites, Zervix- oder Vaginalsekret, Urin und Liquor cerebrospinalis des Subjekts ist.

## Revendications

1. Composition pharmaceutique destinée à être utilisée dans la prévention ou le traitement de septicémie comprenant un inhibiteur de Olfr164 (récepteur odorant 164) :
dans lequel l'inhibiteur de Olfr164 est sélectionné parmi les inhibiteurs d'expression de Olfr164 constitués de : un ARN guide unique (« single guide », ARNsg), un ARN interférent court (« short interfering », ARNsi), un ARN en épingle à cheveux courte (« short hairpin, ARNsh), un microARN (miARN), un ribozyme, et un oligonucléotide antisens, chacun se liant de façon complémentaire à la séquence d'acides nucléiques encodant Olfr164.

2. Composition pharmaceutique destinée à être utilisée de la revendication 1, dans lequel l'inhibiteur de Olfr164 (récepteur odorant 164) est un ARNsg comprenant une séquence nucléotidique ayant au moins 80 %, 85 %, 90 %, ou 95 % d'identité avec SEQ ID n° : 5.

3. Composition pharmaceutique destinée à être utilisée de l'une quelconque des revendications 1 et 2, dans lequel l'inhibiteur de Olfr164 (récepteur odorant 164) est un ARNsg comprenant SEQ ID n° : 5, ou composé de SEQ ID n° : 5.

4. Procédé pour dépister un agent thérapeutique pour la septicémie, comprenant :
(a) le traitement d'un échantillon biologique avec une substance thérapeutique candidate :
(b) la comparaison du niveau d'expression ou de l'activité de Olfr164 entre des échantillons traités avec et sans la substance candidate ; et
(c) la détermination de la substance candidate en tant qu'agent thérapeutique pour la septicémie si le niveau d'expression ou l'activité de Olfr164 dans l'échantillon traité avec la substance candidate est réduit par rapport à l'échantillon traité sans la substance candidate.

5. Procédé de la revendication 4, dans lequel l'échantillon biologique est au moins un sélectionné parmi le groupe constitué de : sang, plasma, sérum, salive, liquide nasal, crachat, liquide synovial, liquide amniotique, ascite, sécrétions cervicales ou vaginales, urine, et liquide cérébrospinal du sujet.
